# EUROPEAN PATENT APPLICATION

(11) **EP 1 408 031 A1**
(43) Date of publication of application: **14.04.2004**
(21) Application number: 02292487.2
(22) Date of filing: 09.10.2002
(51) Int. Cl.: C07D 207/16, A61K 31/40, A61P 31/14

(54) **Pyrolidine derivatives useful in treatment of hepatitis C virus infection**

(71) Applicant: 3 D Gene Pharma, 13006 Marseille (FR)
(72) Inventor: Halfon, Philippe, 13008 Marseille (FR); Sayada, Chalom, 2124 Luxembourg (LU); Feryn, Jean-Marc, 13012 Marseille (FR); Perbost, Régis, 13007 Marseille (FR); Garzino, Frédéric, 13009 Marseille (FR); Camplo, Michel, 13006 Marseille (FR); Courcambeck, Jérôme, 13005 Marseille (FR); Pepe, Gérard, 13600 La ciotat (FR)
(74) Representative: Tezier Herman, Béatrice

(57) **Abstract**

The present invention relates to novel hepatitis C virus ("HCV") protease inhibitors or other flavivirus protease inhibitors of formula, 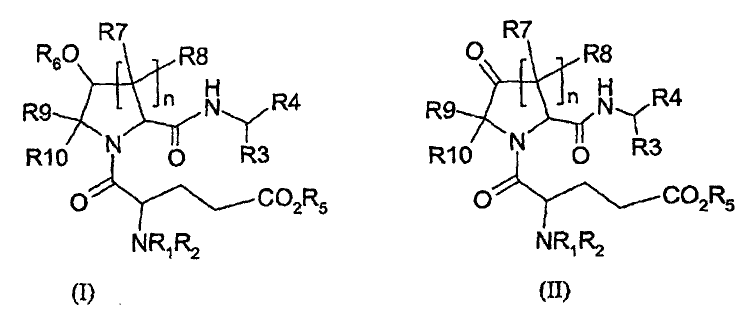 pharmaceutical compositions containing one or more such inhibitors, methods for preparing such inhibitors, uses of these compounds to treat hepatitis C and related disorders together with their use for their activity towards NS3 serine protease, intermediary compounds for the method of preparation of said compounds and screening methods. The invention specifically discloses novel chemical compounds as inhibitors of the HCV serine protease.

## Description

The present invention relates to novel hepatitis C virus ("HCV") NS3 serine protease inhibitors or other flavivirus protease inhibitors, pharmaceutical compositions containing one or more such inhibitors, methods for preparing such inhibitors, uses of these compounds to treat hepatitis C and related disorders together with their use for their activity towards NS3 protease, intermediary compounds for the method of preparation of said compounds and screening methods. The invention specifically discloses novel chemical compounds as inhibitors of the NS3 protease.

### SCIENTIFIC BACKGROUND

Hepatitis C virus (HCV) is a (+)-sense single-stranded RNA virus that has been implicated as the major causative agent in non-A, non-B hepatitis (NANBH), particularly in blood-associated NANBH (BB-NANBH) (see, International Patent Application Publication No. WO 89/04669 and European Patent Application Publication No. EP 381 216).

HCV has been implicated in cirrhosis of the liver and in induction of hepatocellular carcinoma. The prognosis for patients suffering from HCV infection is currently poor. HCV infection is more difficult to treat than other forms of hepatitis due to the lack of immunity or remission associated with HCV infection.

Current data indicates a less than 50% survival rate at four years post cirrhosis diagnosis. Patients diagnosed with localized resectable hepatocellular carcinoma have a five-year survival rate of 10-30%, whereas those with localized unresectable hepatocellular carcinoma have a five-year survival rate of less than 1%.

### Hepatitis C infection

Hepatitis C has emerged in recent years as a common cause of liver disease with an estimated 170-million infected people worldwide. Hepatitis C virus (HCV) infection is characterized by viral persistence and chronic liver disease in approximately 80% of the reported cases. Complications of chronic hepatitis C include cirrhosis in 20% of cases, as well as hepatocellular carcinoma, the incidence of which reaches 4% to 5% per year in patients with cirrhosis. Hepatitis C-related end-stage liver disease is now the principal indication for liver transplantation in industrialized countries (1).

### Brief summary on HCV biology

HCV is a single-strand positive-sense RNA virus which belongs to the *Flaviviridae* family including also yellow fever virus and dengue virus. Upon translation of its unique open reading frame, a single polyprotein or polypeptide is synthesized. This polypeptide is then cleaved by both host and viral proteases to form structural proteins (2).

As this time, the mechanisms of HCV replication in infected cells remain poorly known. It is thought that the RNA-dependent RNA polymerase (RdRp), along with other non structural proteins, and the HCV RNA template and host cell factors, form a ribonucleoproteinic complex of replication wherein perinuclear membranous structures are also associated. Said structures appear to be the site of HCV RNA replication (3-4). By analogy with other *Flaviviridae,* the strategy for replication within this complex seems to be as follows. A (-) strand copy of the RNA genome is produced, serving in turn as a template for the production of a (+) strand RNA. Indeed, said (-) strand HCV RNA was detected in various cells or tissues harboring HCV replication (5-7).

The HCV RdRp, like other viral RNA polymerases, has a high error rate, with misincorporation frequencies averaging about 10⁻⁴ to 10⁻⁵ per base site in the absence of any proofreading mechanism. Consequently, mutations continuously accumulate in newly-generated HCV genomes during replication. Most mutant viral particles are replication deficient, but some can propagate efficiently. The fittest infectious particles are selected continuously on the basis of their replication capacities and under the selective pressure resulting from the environment, said pressure being mainly generated by the immune response of infected patients. This accounts for the presence, in each infected individual, of a pool of genetically-distinct but closely-related HCV variants, collectively referred to as a "quasispecies" (8, 9).

The HCV non structural (NS) proteins are presumed to provide the essential catalytic machinery for viral replication. The NS proteins are derived by proteolytic cleavage of the polyprotein. NS3 is an approximately 68 kDa protein, encoded by approximately 1893 nucleotides of the HCV genome, and has two distinct domains: (a) a serine protease domain consisting of approximately 200 of the N-terminal amino acids; and (b) an RNA-dependent ATPase domain at the C-terminus of the protein. The NS3 protease is considered as a member of the chymotrypsin family because of similarities in protein sequence, overall three-dimensional structure and mechanism of catalysis. Other chymotrypsin-like enzymes are elastase, factor Xa, thrombin, trypsin, plasmin, urokinase, tPA and PSA. The HCV NS protein 3 (NS3) contains a serine protease activity that helps process the majority of the viral enzymes, and is thus considered essential for viral replication and infectivity. It is known that mutations in the yellow fever virus NS3 protease decreases viral infectivity. The first 181 amino acids of NS3 (residues 1027-1207 of the viral polyprotein) have been shown to contain the serine protease domain of NS3 that processes all four downstream sites of the HCV polyprotein. The HCV NS3 serine protease and other associated cofactor, NS4A or NS4B, helps process all of the viral enzymes, and is thus considered essential for viral replication. The HCV NS3 serine protease is responsible for proteolysis of the polypeptide (polyprotein) at the NS3/NS4a, NS4a/NS4b, NS4b/NS5a and NS5a/NS5b junctions and is thus responsible for generating four viral proteins during viral replication. Recently, it is has been revealed that NS3, NS4B and NS5B can interact to form a regulatory complex that could feature in the process of HCV replication (13).

This has made the HCV NS3 serine protease an attractive target for antiviral chemotherapy.

### Hepatitis C current treatments and the need for future therapeutic developments

Treatment of chronic hepatitis C is currently based on the use of recombinant interferon-α (INF-α). INF-α can be administered three times per week subcutaneously, or, when pegylated, once weekly. The antiviral effects of the various forms of IFN-α are enhanced by the addition of ribavirin, a nucleoside analog, the mechanism of action of which remains unclear (1, 10, 11). Currently, the combination of pegylated IFN-α and ribavirin for 24 to 48 weeks leads to definitive viral clearance in 52% to 57% of cases, whereas the remaining patients keep ongoing replication and remain exposed to disease development. These therapies suffer from a low sustained response rate and frequent side effects.

Moreover, no vaccine is available for HCV infection.

Hence, it is generally acknowledged that there is a strong and urgent need for new and highly active anti-HCV molecules. In this respect, the viral protease such the NS3A protease represent putative interesting targets.

WO 02/08244 describes compounds which are deemed to be active for inhibiting HCV NS3 serine protease activity.

### The need for experimental models for HCV studies

HCV exhibits a rather strict species specificity, the infection capacity of which being restricted to humans. At this time, there is no reliable animal model, except for the experimentally-infected Chimpanzee and another primate called Tupaia. Up to recently, there was neither reliable cell culture model for *in vitro* studies of HCV virus. This lack of experimental models has considerably hampered the research on biological properties of HCV, as well as on new therapeutic solutions for HCV infection treatment.

A new long-term, culture system was recently developed (12). According to this *in vitro* model system, human hepatocytes can be cultured for more than one month with a full maintenance of highly differentiated liver functions including: 1) production of plasma proteins such as albumine, alpha-1 antitrypsin, fibrinogen, coagulation factors; 2) production of urea; 3) production of apolipoproteines such as ApoA and ApoB100; 4) response to cytokines including interleukin-1, interleukin-6 and interferon; 5) expression and inducibility of cytochromes P450 gene superfamily; 6) capacity to metabolize drugs and other xenobiotics; and 7) expression and DNA-binding activity of liver-enriched transcription factors such as C/EBP family. Thus, this *in vitro* model appears to be useful as a system for investigating HCV infection (for further details, see reference 12 and example 18).

There is a need for new treatments and therapies for HCV infection and related infections. It is therefore an object of the present invention to provide chemical compounds useful in the treatment or prevention or amelioration of one or more symptoms of hepatitis C.

The inventors have now discovered that some new compounds might modulate the activity of NS3 serine protease.

### SUMMARY OF THE INVENTION

The invention provides a novel class of chemical compounds. The same are useful as inhibitors of serine protease, in particular inhibitors of the HCV NS3 serine protease, it is a further object to provide compositions containing at least one of these compounds useful for the treatment or prevention or amelioration of diseases related or due to an infection by a virus (flavivirus, such as Deng virus, Yellow fever virus and HCV), bacteria or pathogen dependent upon a serine protease for proliferation. A particular embodiment is to provide compositions containing at least one of these compounds useful for the treatment or prevention or amelioration of one or more symptoms of hepatitis C. Another object is to provide methods of preparing pharmaceutical formulations comprising at least one of these compounds. It is a further object to provide methods for preparing the compounds. It is a further object to provide the use of these compounds for the preparation of compositions useful in the treatment, the prevention of the amelioration of diseases related or due to an infection by a virus (flavivirus, such as Deng virus, Yellow fever virus and HCV), bacteria or pathogen dependent upon a serine protease for proliferation. These composition are in particular useful in the treatment, the prevention of the amelioration of one or more of the symptoms of hepatitis C. The invention also provides methods of evaluating the modulation properties of compounds towards NS3 protease.

A further object of the invention is to provide methods for modulating the activity of HCV, particularly of the HCV NS3 protease, using the compounds of the invention.

An other object herein is to provide *in vitro* model useful for investigating HCV infection and particularly the process of HCV NS3 protease.

Among the chemical compounds of the invention, compounds that inhibit HCV NS3 serine protease are preferred.

The present invention discloses compounds having the general structure (I) or (II) as follows : 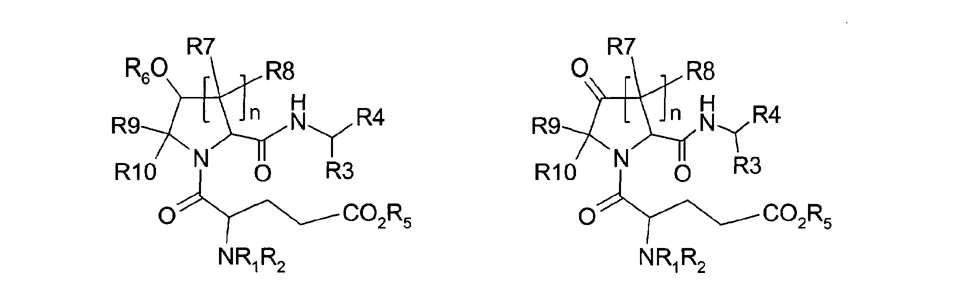 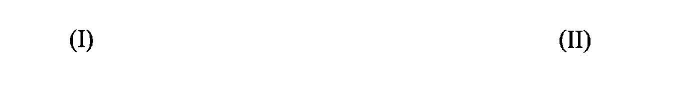 wherein R1 represents a hydrogen atom; R2 represents an amine protecting group; R3 represents an alkyl group having from 1 to 10 carbon atoms inclusive or an amino acid side chain; R4 represents an acid group, an ester group, an alkanoyl group, a keto-acid, a keto-ester, a keto-amide or a α-hydroxy-keto derivative; R5 represents a hydrogen atom, an alkyl group having from 1 to 10 carbon atoms inclusive or a carboxy protecting group; R6 represents a hydrogen atom or an alkyl group having from 1 to 10 carbon atoms inclusive; R7 and R8 are the same or different and each represents a hydrogen atom, an alkyl group having from 1 to 10 carbon atoms inclusive or a cycloalkyl group having from 1 to 10 carbon atoms inclusive, including a cyclohexyl derivative; R9 and R10 are the same or different and each represents a hydrogen atom or an alkyl group having from 1 to 10 carbon atoms inclusive; n is 1 or 2,
their tautomers, optical and geometrical isomers, racemates, salts, hydrates and mixtures thereof.

The compounds of the present invention may have one or more asymmetric centers and it is intended that stereoisomers (optical isomers), as separated, pure or partially purified stereoisomers or racemic mixtures thereof are included in the scope of the invention.

In particular, the preferred compounds are the following isomers :

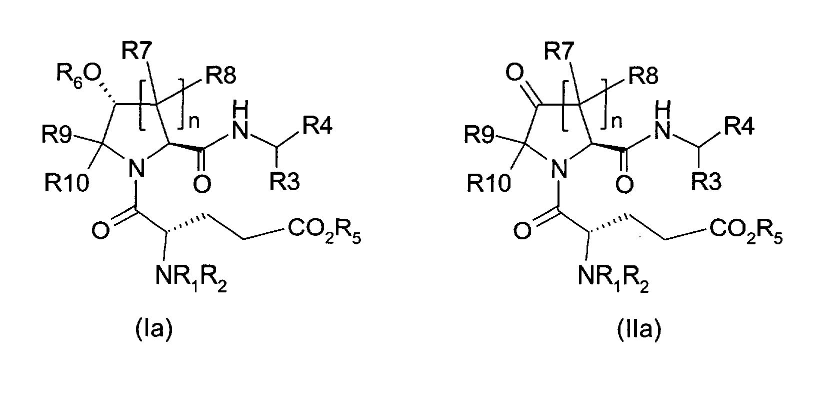

Within the context of the present application, the terms alkyl, alone or in combination with any other term, denote linear or branched, saturated or unsaturated, groups containing from 1 to 10 carbon atoms, preferably from 1 to 6 carbon atoms. An alkanoyl group is a -CO-alkyl group, the alkyl group being as defined above. An alkoxy group is a -O-alkyl group, the alkyl group being as defined above.

The alkyl groups may be linear or branched. Examples of saturated alkyl groups having from 1 to 10 carbon atoms inclusive are methyl, ethyl, propyl, isopropyl, t-butyl, n-butyl, pentyl, hexyl, heptyl, octyl, nonyl, decyl, 2-ethylhexyl, 2-methylbutyl, 2-methylpentyl, 1-methylhexyl, 3-methylheptyl and the other isomeric forms thereof. Preferably, the alkyl groups have from 1 to 6 carbon atoms. The unsaturated alkyl groups having from 2 to 10 carbon atoms include the alkenyl groups. Among the alkenyl groups, a radical containing from 2 to 10 carbon atoms; particularly 2 to 6 carbon atoms, and having one or more ethylenic unsaturations, such as the allyl radical, is preferred.

The term "protecting" refers to when the designated functional group is attached to a suitable chemical group (protecting group).
Examples of suitable amino protecting groups are described in Greene and Wuts, Protective Group in Organic Synthesis, 3^{rd} edition, John Wiley & Sons, New York 1991 and are exemplified in certain of the specific compounds used in this invention. It includes the allyloxycarbonyl (Alloc), benzyloxycarbonyl (Cbz), chlorobenzyloxycarbonyl, t-butyloxycarbonyl (Boc), fluorenylmethoxycarbonyl (Fmoc), isonicotinyloxycarbonyl (I-Noc) groups.
The carboxy protective groups are known in the art (e.g Greene and Wuts, Protective Group in Organic Synthesis) and include aryl (such as the benzyl group : Bn), allyl, or alkyl group.

The term "cycloalkyl", alone or in combination with any other term, refers to a stable non-aromatic 3- to 8-membered carbon ring radical which is saturated and which may be optionally fused, for example benzofused, with one to three cycloalkyl, aromatic, heterocyclic or heteroaromatic rings. The cycloalkyl may be attached at any endocyclic carbon atom which results in a stable structure. Preferred carbocycles have 5 to 6 carbons. Examples of carbocycle radicals include, but are not limited to, cyclopropyl, cyclobutyl, cyclopentyl, cyclohexyl, cycloheptyl, cyclopentyl, cyclohexyl, indane, tetrahydronaphthalene and the like.

The amino acid side chain includes any side chain of the naturally occurring (L form) or synthesized (L or D form) alpha-aminoacids. In particular, the side chain may represent -CH3, -CH(CH3)2, -CH2-CH(CH3)2, -CH(CH3)C2H5, H, -CH2OH, - CH(OH)CH3, -CH2SH, -(CH2)2-S-CH3, -CH2C6H5, -CH2-C6H5(OH), -CH2CONH2, -(CH2)2CONH2, -CH2COOH, -(CH2)2COOH, -(CH2)4NH2, -(CH2)3NHC(NH2)2, etc. Preferably, the amino acid side chain is -CH2-CH(CH3)2 or -CH3, optionally substituted, particularly with a trifluoro(C1-C6)alkyl group, such as -CF3.

Any of these groups or derivatives, including the amino acid side chain, may be optionally substituted with one or more groups selected from hydroxyl group, halogen atom (including F, Cl or Br), cyano group, nitro group, acid group, ester (-COO(C1-C6)alkyl group), -OCO(C1-C6)alkyl group, amide (-NHCO(C1-C6)alkyl or -CONH(C1-C6)alkyl group), (C1-C10)alkyl radical, (C1-C10)alkoxy radical, mono-or poly-cyclic hydrocarbon group, C=O group, an amino group or a trifluoro(C1-C6)alkyl group.

The term mono- or poly-cyclic hydrocarbon group is understood to refer to hydrocarbon cyclic group having from 1 to 20 carbon atoms, optionally interrupted with one or more heteroatoms selected in the group N, O , S and P. Among such mono- or poly-cyclic hydrocarbon groups, cyclopentyl, cyclohexyl, cycloheptyl, 1-or 2-adamantyl groups, pyran, piperidine, pyrrolidine, morpholine, dioxan, tetrahydrothiophene, and tetrahydrofuran can be cited.

Preferably, the amino group is preferably NH2, NHCH3, or NHC2H5.

The trifluoro(C₁-C₆)alkyl group is preferably the trifluoromethyl group.

### PREFERRED EMBODIMENTS

According to the present invention, the compounds correspond to the general formula (I) or (II), with R4 which represents an acid group (-COOH), an ester group (-COOR), an alkanoyl group (-COR), a keto-acid (-COCOOH), a keto-ester (-COCOOR), a keto-amide (-COCONHR) or a α-hydroxy-keto (-CHOHCOR) derivative, wherein R independently represents a hydrogen atom, a hydroxyl radical, an alkyl group, an alkoxy group or an amino group as defined above. Where R4 represents -COOR, -COCOOR, -COR or -COCONHR group, R is preferably an alkyl group (in particular a methyl or ethyl radical). Where R4 is -CHOHCOR, R is preferably a hydroxyl radical, an alkoxy group (in particular methoxy or ethoxy), or an amino group (in particular NH2, NHCH3, or NHC2H5).

According to preferred embodiments, the compounds according to the invention correspond to general formula (I) or (II) wherein :
R2 stands for benzyloxycarbonyl (Cbz) or t-butyloxycarbonyl (Boc) groups and/or R3 stands for -CH₂-CF₃ or -CH₂-CHMe₂; and/or R4 is acid, -CHOHCOR, with R is OH or an alkoxy group (preferably methoxy or ethoxy), keto-acid, keto-ester (preferably -COCOOMe or-COCOOEt) ; and/or R5 is H or benzyl ; and/or R6 stands for t-butyl ; and/or R7 is H ; and/or R8 is H ; and/or R9 is H; and/or R10 is H and/or n = 1.

A particular preferred group of compounds according to the present invention, are compounds of formula (I).

Specific examples of compounds of formula (I) or (II) which fall within the scope of the present invention include the following compounds :

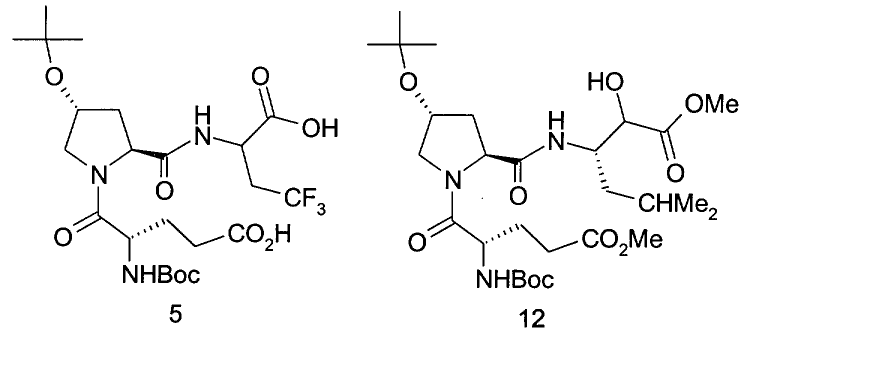

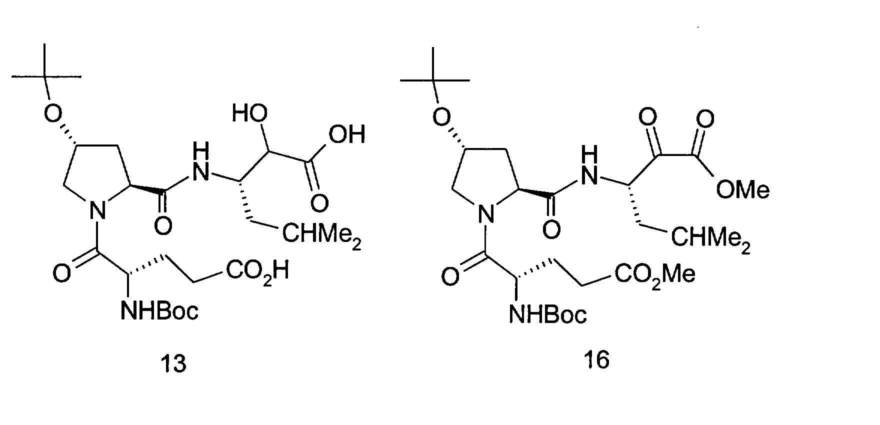

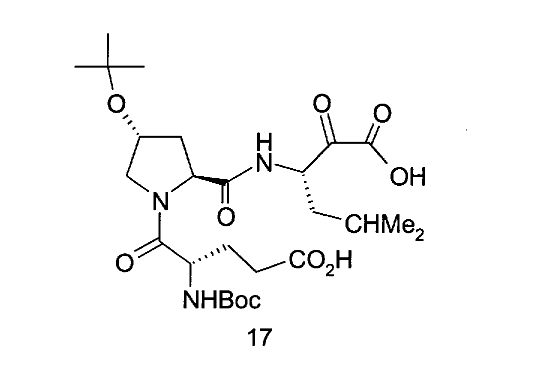

The compounds of the present invention present the advantage to be non bactericidal and/or non mutagenic. The ames test with four different strains, with or without metabolic activation, has been carried out with the above cited compounds (in particular, compound 5). The results thereof have shown that the compounds above identified (in particular, compound 5) are non mutagenic.

As stated earlier, the invention includes also tautomers, rotamers, enantiomers and other stereoisomers of the inventive compounds. Thus, as one skilled in the art appreciates, some of the inventive compounds may exist in suitable isomeric forms. Such variations are contemplated to be within the scope of the invention.

As used herein, the compounds of this invention, including the compounds of formula (I) or (II), are defined to include pharmaceutically acceptable derivatives or prodrugs thereof. A "pharmaceutically acceptable derivative or prodrug" means any pharmaceutically acceptable salt, ester, salt of an ester, or other derivative of a compound of this invention which, upon administration to a recipient, is capable of providing (directly or indirectly) a compound of this invention.

Accordingly, this invention also provides prodrugs of the compounds of this invention, which are derivatives that are designed to enhance biological properties such as oral absorption, clearance, metabolism or compartmental distribution. Such derivations are well known in the art. As the skilled practitioner realizes, the compounds of this invention may be modified by appending appropriate functionalities to enhance selective biological properties. Such modifications are known in the art and include those which increase biological penetration into a given biological compartment (e.g., blood, lymphatic system, central nervous system), increase oral availability, increase solubility to allow administration by injection, alter metabolism and alter rate of excretion.

Particularly favored derivatives and prodrugs are those that increase the bioavailability of the compounds of this invention when such compounds are administered to a mammal (e.g., by allowing an orally administered compound to be more readily absorbed into the blood), have more favorable clearance rates or metabolic profiles, or which enhance delivery of the parent compound to a biological compartment (e.g., the brain or lymphatic system) relative to the parent species. Preferred prodrugs include derivatives where a group which enhances aqueous solubility or active transport through the gut membrane is appended to the structure of formula (I).

Bioavailability refers to the rate and extent to which the active drug ingredient or therapeutic moiety is absorbed into the systemic circulation from an administered dosage form as compared to a standard or control.

The compounds of the present invention may include salts thereof. When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

The pharmaceutically acceptable salts are prepared by reacting the compound of formula I or II with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol, etc. Mixture of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, fonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane, etc. Mixture of solvents may also be used.

In another embodiment, this invention provides a pharmaceutical composition comprising at least one compound as defined above. The pharmaceutical composition generally additionally comprises a pharmaceutically acceptable vehicle or carrier. Because of their HCV inhibitory activity; such pharmaceutical compositions possess utility in treating hepatitis C and related disorders.

In yet another embodiment, the present invention discloses methods for preparing pharmaceutical compositions comprising the inventive compounds as an active ingredient.

In the pharmaceutical compositions and methods of the present invention, the active ingredients will typically be administered in admixture with suitable carrier materials suitably selected with respect to the intended form and administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices.

The compounds may thus be formulated in various forms, including solid and liquid forms, such as tablets, capsules, gel, syrup, powder, aerosol, suppositories etc.

The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.

Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.

Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

Additionally, the compositions of the present invention may be formulated in a sustained release form to provide the rate controlled release of any one or more of the components or active ingredients to optimize the therapeutic effects, i.e. HCV inhibitory activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and pacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for either oral or parental administration. Such liquid forms include solutions, suspensions and emulsions.

The compounds of the invention may also be deliverable transdermally. The transdermal compositions may take the form of creams, lotions, aerosols and/or emulsions and can be included in the transdermal patch of the matrix or reservoir type as are conventional in the art for this purpose.

Preferably, the compounds are administered orally, intravenously or subcutaneously.

The dosages and dosage regimen in which the compounds of formula (I) are administered will vary according to the dosage form, mode of administration, the condition being treated and particulars of the patient being treated. Accordingly, optimal therapeutic concentrations will be best determined at the time and place through routine experimentation.

The compounds according to the invention can be used enterally or parenterally. Orally, the compounds according to the invention are suitably administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules. A preferred method of administration consists in using a suitable form containing from 1 mg to about 500 mg of active substance.

The compounds according to the invention can also be administered parenterally in the form of solutions or suspensions for intravenous or intramuscular perfusions or injections. In that case, the compounds according to the invention are generally administered at the rate of about 10 µg to 10 mg per day per kg of body weight; a preferred method of administration consists of using solutions or suspensions containing approximately from 0.01 mg to 1 mg of active substance per ml.

The compounds of the present invention can be used in a substantially similar manner to other known anti-hepatitis C agents. For the compounds of this invention, the anti-hepatitis C dose to be administered, whether a single dose, multiple dose, or a daily dose, will of course vary with the particular compound employed because of the varying potency of the compound, the chosen route of administration, the size of the recipient, and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. Someone skilled in the art of hepatitis C treatment will be able to ascertain, without undue experimentation, appropriate protocols for the effective administration of the compounds of this present invention, such as by referring to the earlier published studies on compounds found to have anti-hepatitis C properties.

Preferably, the pharmaceutical preparation is in a unit dosage form. In such form, the preparation is subdivided into suitable sized unit doses containing appropriate quantities of the active components, e.g., an effective amount to achieve the desired purpose.

The quantity of the inventive active compound in a unit dose of preparation may be generally varied and adjusted from about 0.2 milligrams to about 1000 milligrams, preferably from about 0.5 to about 950 milligrams, more preferably from about 1.0 to about 500 milligrams, and typically from about 1 to 250 milligrams, according to the particular application. The actual dosage employed may be varied depending upon the patient's age, sex, weight and severity of the condition being treated. Such techniques are well known to those skilled in the art.

Generally, the human oral dosage form containing the active ingredients can be administered 1 or 2 times per day. The amount and frequency of the administration will be regulated according to the judgment of the attending clinician. A generally recommended daily dosage for oral administration may range from about 1.0 milligram to about 1,000 milligrams per day, in single or divided doses.

Conventional methods for preparing tablets are known. Such methods include dry methods such as direct compression and compression produced by compaction, or wet methods or other special procedures. Conventional methods for making other forms for administration such as, for example, capsules, suppositories and the like are also well known.

The compounds of the invention can be used alone or in combination with immunomodulatory agents, such as interferons, in particular α-interferon; other antiviral agents such as ribavirin and amantadine; other inhibitors of hepatitic C protease; inhibitors of other targets in the HCV life cycle including the helicase, polymerase, metalloprotease, or internal ribosome entry; or combinations thereof. The other active ingredient can be administered in combination with a compound of the present invention either simultaneously, separately, or sequentially.

By "simultaneous", it is meant that the two compounds are administered at the same time, though not necessarily in the same composition. By "sequential", it is meant that the two compounds are administered within a time period such that the first of the two compounds is still active in the patient when administration of the second of the two compounds occurs.

Preferably, "sequential" means within the same 24 hour, preferably within the same 12 hour, such as within the same 6, 3,1, half or quarter hour time period.

The present invention provides compounds that are useful as serine protease inhibitors, in particular as viruses (preferably flavivirus, such as dengue virus, Yellow fever virus and HCV), bacteria or any other pathogens serine protease inhibitors, and more preferably as HCV NS3 protease inhibitors. As such, they act by interfering with the life cycle of the HCV virus and any other virus, bacteria or pathogen that are dependent upon a serine protease for proliferation and/or infection. Preferably, these compounds are useful as antiviral agents.

Another embodiment of the invention discloses the use of the pharmaceutical compositions disclosed above for treatment of diseases, in particular for treating diseases related or due to an infection by a virus (preferably flavivirus, such as dengue virus, Yellow fever virus and HCV), bacteria or pathogen dependent upon a serine protease for proliferation, more particularly for treating HCV infection and the like. The method comprises administering a therapeutically effective amount of the inventive pharmaceutical composition to a mammal (in particular a patient) having such a disease or diseases and in need of such a treatment.

The present invention relates to a method for the treatment of a disease, and in particular a disease related or due to an infection by a virus (preferably flavivirus, such as dengue virus, yellow fever virus and HCV), bacteria or pathogen dependent upon a serine protease for proliferation. More particularly, the present invention relates to a method to treat HCV infection and the like, such as the complications of hepatitis C, in particular chronic hepatitis, cirrhosis, hepatocellular carcinoma, or extra hepatic manifestation, comprising administering to a mammal (in particular a patient) in need of such treatment an effective amount of at least one compound of the present invention as defined herein.

A further object of this invention is the use of an effective amount of at least one compound of formula (I) or (II) as defined above for the preparation of pharmaceutical composition for the treatment of a disease associated or due to an infection by a virus (preferably flavivirus, such as dengue virus, Yellow fever virus and HCV), bacteria or pathogen dependent upon a serine protease for proliferation. More particularly, the pharmaceutical composition is intended to treat a disease associated with HCV infection.

Because of their anti-pathogen properties and particularly their HCV inhibitory activity, the compounds of this invention are suitable for treating a variety of diseases in a variety of conditions. In this regard, "treatment" or "treating" include both therapeutic and prophylactic treatments. Accordingly, the compounds may be used at very early stages of a disease, or before early onset, or after significant progression.

Typical examples of diseases associated with HCV infection include chronic hepatitis, cirrhosis, hepatocarcinoma, or extra hepatic manifestation for instance. The compounds of this invention are particularly suited for the treatment of hepatitis C and complications thereof, in particular cirrhosis or hepatocellular carcinoma.

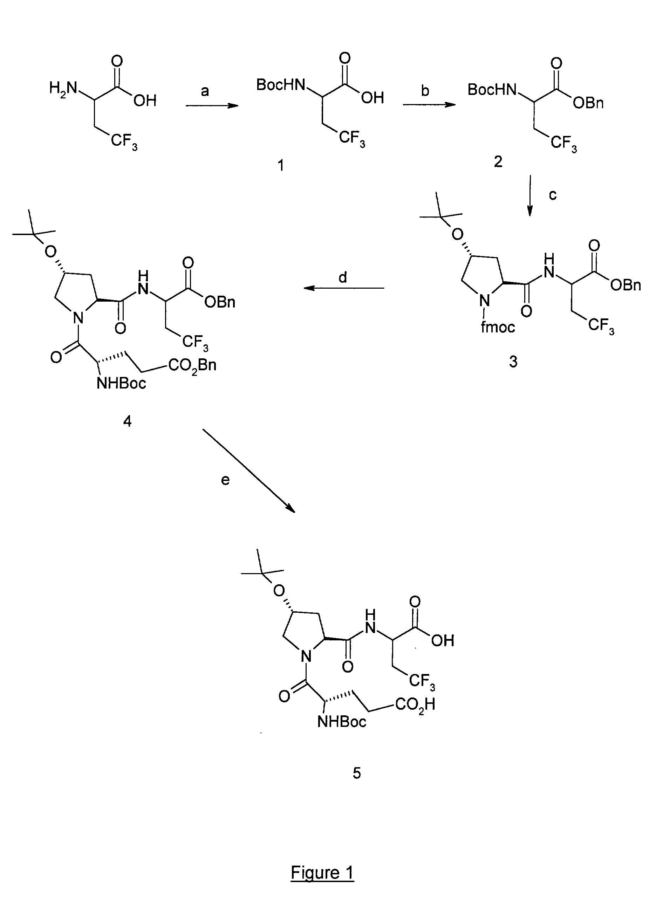

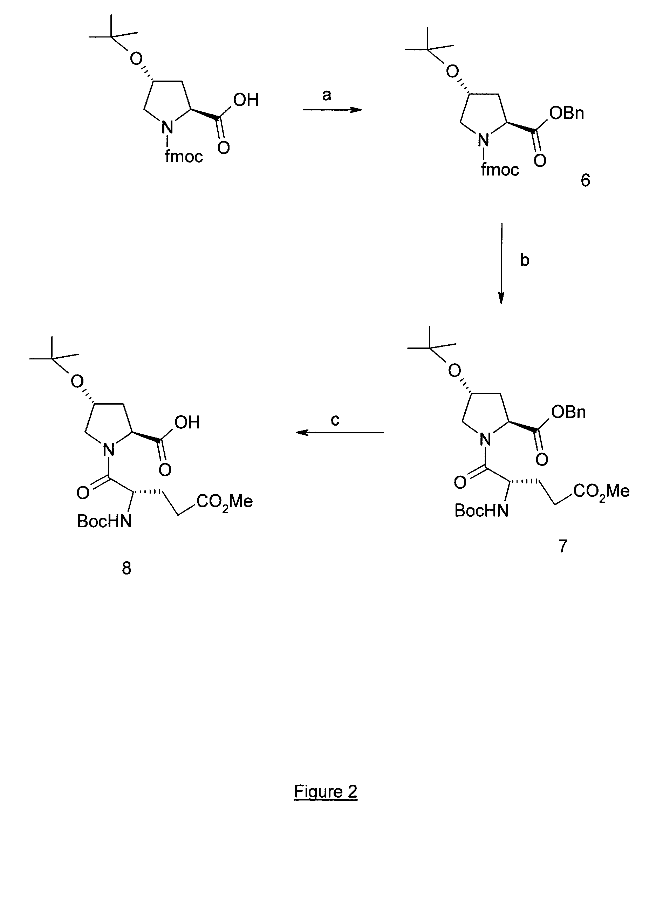

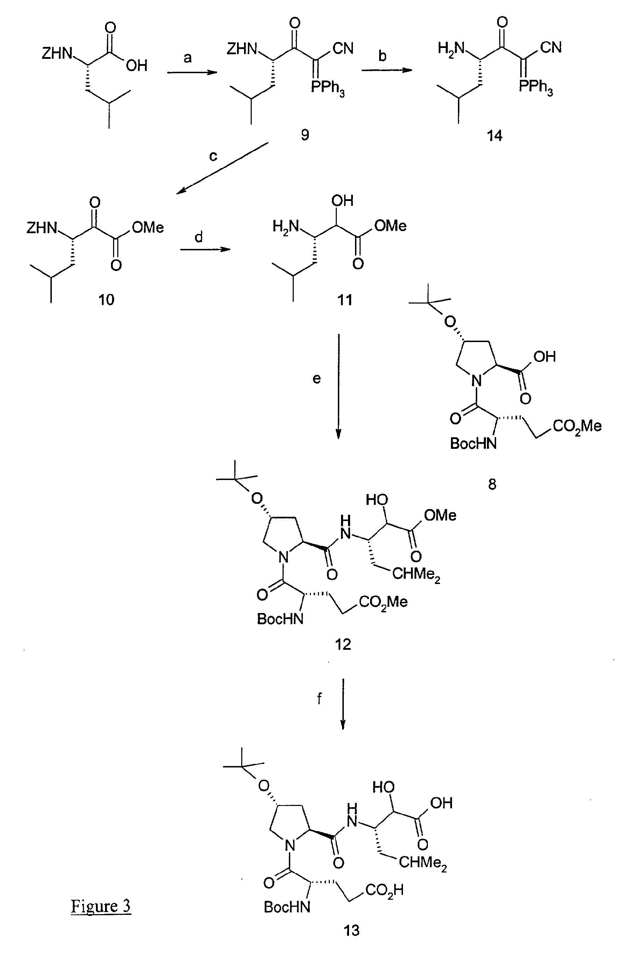

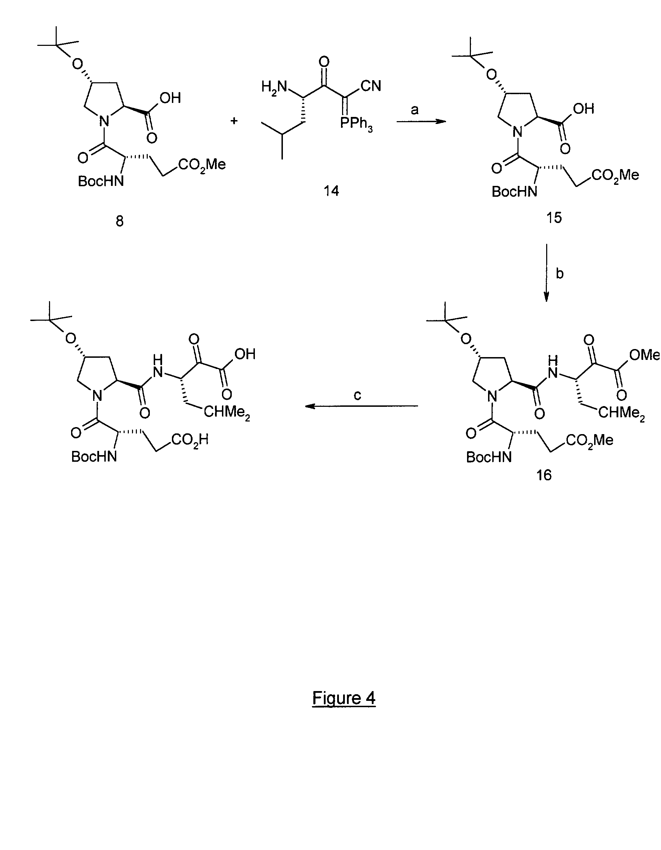

Another embodiment of the invention discloses a method of preparing the compounds disclosed herein. The compounds may be prepared by several techniques known in the art. Representative illustrative procedures are outlined in the following reaction schemes (Figures 1-4). It is to be understood that while the following illustrative schemes describe the preparation of a few representative inventive compounds, suitable substitutions of any substituant will result in the formation of the desired compounds based on such substitution. Such variations are contemplated to be within the scope of the invention.
In particular, they can be prepared by the following general method(s) :
Compounds of general formula (I) and (II) can be obtained by general peptide coupling methods of a central proline derivative with usual or modified amino acids derivatives. The reactants used in these procedures are standard peptide coupling reagents like BOP, PyBOP, DCC, HOBT, etc. The conditions are mainly in usual organic solvents, such as ethylacetate (EtOAc), dichloromethane (DCM) , dichloroethane (DCE), dimethylformamide (DMF) and usually at room temperature (18-25°C).
General Strategy : A proline moiety including specific modifications was firstly prepared before to be C-terminal coupled to a modified amino-acid (including ketone, ketoacid or ketoester derivatives). Following the standard N-deprotection of the resulting compound, the last synthon (N-protected amino acid derivative) was finally added to provide an intermediate compound which finally provides after standard deprotection steps the target molecule (compound of the present invention).

Following the above described methods for preparing the compounds of formula (I) or (II), the present invention further provides compounds corresponding to the following formula (III) : 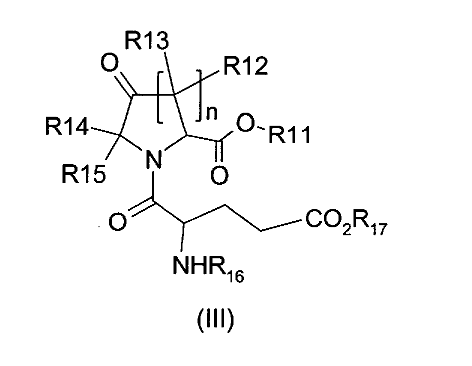 wherein R12, R13, R14 and R15, independently from each other, represents a hydrogen atom, an alkyl, an alkoxy group, a cycloalkyl, an acid group, an ester group, an alkanoyl group, a keto-acid, a keto-ester, a keto-amide or a α-hydroxy-keto group; R16 represents an amine protecting group; R11 and R17, independently from each other, represents a hydrogen atom, an alkyl group or a cycloalkyl group, including cyclohexyl group; and n is 1 or 2, their tautomers, optical and geometrical isomers, racemates, salts, hydrates and mixtures thereof.

The above cited groups are as defined and specified for compounds of formulae (I) and (II). They can also be substituted as mentioned above.
The compounds of formula (III) may have one or more asymmetric centers and it is intended that stereoisomers (optical isomers), as separated, pure or partially purified stereoisomers or racemic mixtures thereof are included in the scope of the invention.

In particular, the preferred compounds of formula (III) are the following isomers :

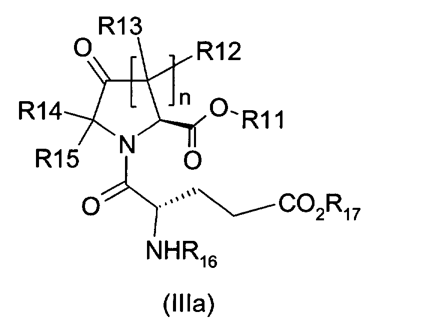

According to a specific embodiment, compounds of formula (III) correspond to compounds of formula (III) wherein R12, R13, R14 and R15, independently from each other, represents a hydrogen atom, an alkyl, an alkoxy group, or a cycloalkyl group, and preferably a hydrogen atom.

Specific examples of compounds of formula (III) which fall within the scope of the present invention include the following compound :

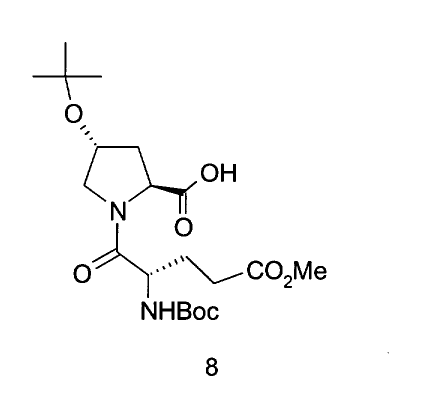

According to another specific embodiment, compounds of formula (III) correspond to compounds of formula (III) wherein at least one of R12, R13, R14 and R15 represents an acid group, an ester group, an alkanoyl group, a keto-acid, a keto-ester, a keto-amide or a α-hydroxy-keto group, as defined above.

Compounds of formula (III) may be used either as intermediate compounds to prepare compounds of formula (I) or (II) or as active ingredients, such as the compounds of formula (I) and (II) of the present invention and in particular as antiviral agents, such as antiviral HCV agent.

### NS3 Inhibitory Activity

Primary cultures of normal human hepatocytes infected in vitro and supporting sustained HCV replication, the closest model to the *in vivo* HCV-infected human liver, were used to show that the compounds of the invention are able to block HCV replication in infected human hepatocytes. In view of these results, said compounds appear to be primarily involved through their nonspecific antiviral effect. The primary cultures of human hepatocytes described herein provide a good *in vitro* model for studying intrinsic HCV resistance to the antiviral properties of these compounds, and for screening the potential therapeutic capacity of new molecules, e.g., the chemical compounds of the invention, to inhibit HCV infection and/or replication.

In an alternate embodiment, this invention provides a method of evaluating the modulation properties of compounds towards NS3 serine protease, particularly HCV NS3 serine protease, said methods implementing *in vitro* primary cultures of human hepatocytes described herein. In this respect, the present invention provides a method for screening and/or characterizing compounds that present antiviral activity, in particular antiviral HCV activity, by implementing *in vitro* primary cultures of human hepatocytes described herein. In particular said methods comprise:
a) contacting a test compound with the *in vitro* primary cultures of human hepatocytes in presence of HCV or active part thereof, and
b) determining the antiviral activity of the test compound in comparison with the antiviral activity of one of the compounds of the present invention.
The *in vitro* primary cultures of human hepatocytes is more specifically described in reference 12, which is incorporated herein by reference.

More particularly, the *in vitro* primary cultures of human hepatocytes were prepared as follows : hepatocytes were prepared from liver tissue resected from donors who tested negative for HCV, and inoculation was performed 3 days after plating with 33 HCV serum samples of different virus load and genotype. The presence of intracellular HCV RNA, detected by a strand-specific rTth RT-PCR assay, was used as evidence of infection. A kinetics analysis of HCV replication revealed that intracellular negative-strand RNA appeared at day 1 post-infection with a maximum level at days 3 and 5, followed by a decrease until day 14. At day 5, the copy level of viral RNA was estimated to be amplified at least 15-fold in infected cells. The level of intracellular HCV RNA in response to different serum samples was reproducible from one hepatocyte culture to another, suggesting that there is no inter-individual variability in the susceptibility of hepatocytes to HCV infection. These findings indicated that adult human hepatocytes in primary culture retain their susceptibility to in vitro HCV infection and support HCV RNA replication. This model represents therefore a valuable tool for the study of initial steps of the HCV replication cycle and for the evaluation of antiviral molecules.

In another embodiment, the invention discloses methods of decreasing protease activity in a mammal comprising the step of administrating to said mammal any of the pharmaceutical compositions and combinations described above. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle. Such additional agent may be administered to the mammal prior to, concurrently with, or following the administration of the HCV inhibitor composition.

In a preferred embodiment, these methods are useful in decreasing HCV NS3 serine protease activity in a mammal. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle such as helicase, polymerase, or metallo protease. Such additional agent may be administered to the mammal prior to, concurrently with, or following the administration of the compositions of this invention.

In an alternate preferred embodiment, these methods are useful for inhibiting viral replication in a mammal. Such methods are useful in treating or preventing, for example, viral diseases, such as HCV. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle.

Such additional agent may be administered to the mammal prior to, concurrently with, or following the administration of the composition according to this invention.

The compounds set forth herein may also be used as laboratory reagents. The compounds of this invention may be used to treat or prevent viral contamination of materials and therefore reduce the risk of viral infection of laboratory or medical personnel or patients who come in contact with such materials. These materials include, but are not limited to, biological materials, such as blood, tissue, etc; surgical instruments and garments; laboratory instruments and garments; and blood collection apparatuses and materials.

Further aspects and advantages of this invention will be disclosed in the following examples, which should be regarded as illustrative and not limiting the scope of this application.

### LEGEND TO THE FIGURES

**Figure 1:** reaction scheme to prepare compound 5. The reactants and/or solvents are as follows : a) Boc2O, Dioxane, NaOH/H2O; b) BnOH, DCC/DMAP; c) TFA, DCE then BOP, DCE, fmoc-L-Hyp(OtBu), DIEA; d) DEA, CH3CN then PyBOP, DIEA, Boc-L-Glu(OBn)OH; e) H2, Pd/C, EtOH
**Figure 2:** reaction scheme to prepare intermediate compound 8. The reactants and/or solvents are as follows : a) BnOH, DCM, DCC, DMAP; b) DEA, CH3CN then DIEA, DCM, PyBOP and Boc-L-Glu(Ome)-OH; c) H2, Pd/C, MeOH
**Figure 3:** reaction scheme to prepare compounds 12 and 13. The reactants and/or solvents are as follows : a) NC-CHPPh3, DCM, EDCI, DMAP; b) Pd/C, HC02NH4, MeOH; c) 03, DCM, MeOH; d) H2, Pd/C; e) DIEA, PyBop, DCM; f) NaOH 1M, MeOH
**Figure 4:** reaction scheme to prepare compounds 16 and 17. The reactants and/or solvents are as follows : a) PyBOP, DIEA, DCM; b) 03, DCM, MeOH; c) NaOH 1 M, MeOH
**Figure 5:** Inhibitory effect of compound of example 5 expressed as the ratio HCV RNA/GAPDH RNA

### EXAMPLES

### Preparation of the compounds.

Nuclear magnetic resonance spectra were recorded on a Bruker WH-250 (250 MHz) spectrometer. The chemical shift values are expressed in ppm (part per million) relative to tetramethylsilane as internal standard; s = singlet; d = doublet, dd = doublet of doublet; t = triplet; q = quartet; m = multiplet; bs = broad singlet. Coupling constants J are expressed in Hz. The relative integrals of peak areas agreed with those expected for the assigned structures. Thin layer chromatography (TLC) was performed on POLYGRAM Sil G/UV 254 silica gel plates with fluorescent indicator and the spots were visualized under 254 and 366 nm illumination. Proportions of solvents used for TLC are by volume. All solvents and most chemicals were purchased from Aldrich Chemical Co. and were used as received. Amino acids were purchased from Neosystem Group SNPE.

### Abbreviations

Boc: t-butyloxycarbonyl, Boc₂O: di-t-butyl-dicarbonate; BOP: Benzotriazol-1-yloxyl-tris-(dimethylamino)-phosphonium hexafluorophosphate; DCC: N,N'-dicyclohexylcarbodiimide; DCE: 1,2- dichloroethane; DCM: dichloromethane; DEA: diethylamine; DIEA: diisopropylethylamine; DMAP: 4-dimethylaminopyridine; EDCI: 1-(3-Dimethylaminopropyl)-3-ethylcarbodiimide hydrochloride; EtOAc: Ethyl Acetate; Fmoc: -(9H-fluoren-9-ylmethoxy)carbonyl; Glu: glutamate, Hyp: hydroxyproline; PyBOP: Benzotriazol-1-yloxyl-tris-(pyrrolidino)-phosphonium hexafluorophosphate; TFA: Trifluoro Acetic Acid, Z: Benzyloxycarbonyl.

### Example 1:

### Compound 1

A solution of 2-amino-4,4,4-trifluorobutyric acid (100mg, 0.636 mmol) in dioxane (0.6 mL) and 1M NaOH_{aq} (1.2 mL) was stirred at 0°C for 10 min. Boc₂O (166 mg. 0.763 mmol) was then added and the mixture was stirred at room temperature for 24h. The pH was periodically adjusted to 9 by addition of a 1 M NaOH_{aq} solution. The mixture was extracted with Et₂O then acidified (pH=2) with the addition of 5% aqueous HCl solution. The acidic aqueous phase was extracted with EtOAc and combined organic phases were washed with brine. After drying on MgSO₄, concentration under reduced pressure afforded the protected amino acid 1 (92 mg, 71%).

### Example 2:

### Compound 2

Compound **1** (54 mg, 0.215 mmol) was suspended at room temperature in DCM (5 mL) with 0.06 mL of benzyl alcohol (0.537 mmol), DCC (44 mg, 0.236 mmol) and DMAP (29 mg, 0.236 mmol). The solution was stirred for 18 h, filtered on Celite and successively washed with a 5% solution of aqueous HCl and a 10% solution of aqueous NaHCO₃. The organic phases were dried over MgSO₄, evaporated and the residue was purified by chromatography (eluent gradient hexane/ether 95:5 to **1:1**) to give the pure compound **2** (41 mg, 55%).
¹H NMR (CDCl₃) δ: 1.34 (s, 9H), 2.65 (m, 2H), 4.50 (m, 1H), 5.10 (s, 2H), 5.18 (m, 1 H), 7.30 (m, 5H).

### Example 3:

### Compound 3

Compound **2** (664mg, 1,91 mmol) was suspended in DCE at room temperature and 10 eq of TFA (1,47 mmol) were added. The solution was stirred for 2 h, evaporated and suspended in DCE (10 mL). Fmoc-L-Hyp(OtBu) (835mg, 2,0 mmol), 2eq of DIEA (665 µL, 3,82 mmol) and 1,06 eq of BOP (897 mg, 2,03 mmol) were added and the mixture was stirred for 20h at room temperature. The solvent was then evaporated and the residue dissolved in EtOAc. The organic phases was successively washed with aqueous solutions of 5% citric acid and 5% NaHCO₃. The organic phases were dried over MgSO₄, evaporated and the residue was purified by chromatography (30% EtOAc/Hexane) to give the pure compound **3** (1,2g, quantitative). This compound was directly used in the next step.

### Example 4:

### Compound 4

Compound 3 (521 mg, 0.81 mmol) was treated with a solution of 10% DEA in CH₃CN and stirred for 20h at room temperature. The mixture was evaporated and dissolved in DMF (9mL). Boc-L-Glu(OBn)OH (300mg, 0.89 mmol), 2eq of DIEA (282 µL, 1.62 mmol) and 1.1 eq of BOP (394 mg, 0.89 mmol) were then added and the mixture was stirred for 24h. The solvent was evaporated and the residue dissolved in EtOAc. The organic phasis was successively washed with aqueous solutions of 5% citric acid and 5% NaHCO₃ and finally dried over MgSO₄. The residue was purified by chromatography (30% EtOAc/Hexane) to give the pure compound **4** (72mg, 12%).
¹H NMR (CDCl₃) δ: 0.3 (s, 9H), 0.5 (s, 9H), 1.9-2.7 (m, 6H), 3.2 (m, 1H), 3.5 (m, 1 H), 4.0-4.5 (m, 3H), 4.9 (s, 4H), 5.0-5.2 (m, 1 H), 7.2 (m, 10H).

### Example 5:

### Compound 5

Compound **4** (74mg, 0.1 mmol) was dissolved in a mixture of 1/3 EtOAc/EtOH containing 20% Pd(OH)₂ (50mg) and stirred under a hydrogen atmosphere for 12h at room temperature. The mixture was then filtered on Celite and evaporated. It was then dissolved in a minimum of Et₂O and few drops of hexane were added. The precipitate was filtered and vacuum dried to give compound **5** (35mg, 63%) as a white powder.
¹H NMR (CDCl₃) δ: 0.3 (s, 9H), 0.5 (s, 9H), 1.0-2.0 (m, 6H), 2.4-3.0 (m, 2H), 3.2-3.8 (m, 3H), 4.6 (m, 1 H), 5.0 (m, 1 H), 6.0 (bs, 1H), 6.9 (bs, 1 H). SM (GT, FAB⁺): 556 (M+1H)⁺

### Example 6:

### Compound 6

Fmoc-L-Hyp(OtBu) (410 mg, 1 mmol) was dissolved in DCM (15 mL). BnOH (1.5 mmol), DCC (1 mmol) and DMAP (0.1 mmol) were then added and stirred for 18h at room temperature. The precipitate was removed by filtration through Celite and the filtrate was washed with 5% aqueous HCl solution followed by saturated NaHCO₃. The organic phase was dried over MgSO₄, concentrated under reduced pressure and purified by flash chromatography (hexane/Et₂O from 95:5 to 1:1) to give compound **6** (300 mg, 60%).
¹H NMR (CDCl₃) δ : 1.36 (s, 9H), 2.30-2.45 (m, 2H), 3.55 (dd, 1H, J=5.0, 10.0), 4.00 (m, 1 H), 4.40-4.60 (m, 2H), 4.70 (m, 1 H), 5.30 (d, 1H, J=2.2), 5.40 (d, 1 H, J=4.8), 7.40-7.60 (m, 10H), 7.60-8.00 (m, 3H).

### Example 7:

### Compound 7

Compound **6** (390 mg, 0.78 mmol) was dissolved in a solution of anhydrous CH₃CN (5 mL) with DEA (600 µL). The mixture was stirred for 3h at room temprature. DCE (10mL) was added an dthe mixture concentrated under reduced pressure. The residue was dissolved in DCE (5mL) and DIEA (271 µL, 1.56 mmol). Boc-L-Glu(OMe)OH.DCHA (380mg, 0.858 mmol) and BOP (379 mg, 0.858 mmol) were added. The mixture was allowed to stirr for 24h at room temperature. It was then washed with a 5% aqueous HCl solution followed by a saturated aqueous NaHCO₃ and finally dried over MgSO₄. Concentration under reduced pressure gave a crude product which was purified by flash chromatography (hexane/EtOAc 90:10 to 70:30) to give **7** (294 mg, 72%).
¹H NMR (CDCl₃) δ : 1.1 (s, 9H), 1.4 (s, 9H), 1.5-1.8 (m, 2H), 2.0-2.2 (m, 2H), 2.25-2.5 (m, 2H), 3.5 (m, 1 H), 3.6 (s, 3H), 3.8 (s, 1 H), 4.25 (m,1 H), 4.4 (m, 1 H), 4.6 (m, 1 H), 5.1 (m, 2H), 5.3 (d, 1 H), 7.2 (s, 5H).
¹³C NMR (CDCl₃) δ : 14.52, 21.31, 28.64, 37.24, 43.76, 51.91, 58.05, 67.19, 69.80, 74.59, 79.88, 128.00, 128.89, 135.92, 155.74, 171.01, 171.98, 173.68.

### Example 8:

### Compound 8

Compound **7** (468 mg, 0.9 mmol) was dissolved in MeOH (25 mL) and 10% Pd on charcoal (400 mg) was added. The mixture was stirred at room temperature under a hydrogen atmosphere for 3h. The catalyst was removed by filtration trough Celite and the filtrate concentrated. The crude acid **8** was immediately used without purification.

### Example 9:

### Compound 9

Z-L-LeuOH (mg, 2.4 mmol) was dissolved in anhydrous DCM (24 mL). EDCI (464 mg, 2.42 mmol) and DMAP (29 mg, 0.242 mmol) were successively added and the mixture was stirred for 30 min. PPh₃CHCN (729 mg, 2.42 mmol) was then added and the mixture was stirred at room temperature for 24h. Reaction was quenched with 2% aqueous HCl solution. The organic phase was then washed with saturated NaHC03 aqueous solution, dried over MgSO₄ and concentrated under reduced pressure. The crude solid was purified by flash chromatography (hexane/EtOAc 9:1 to 1:1) to give compound **9** (1.31 mmol, 55%).
¹H NMR (CDCl₃) δ : 0.72 (d, 1H, J=6.0), 0.78 (d=3H, J=6.0), 0.89 (d, 3H, J=6.00), 1.60 (m, 2H), 4.68 (m, 1 H), 4.90 (m, 1 H), 5.50 (s, 2H), 7.30-7.65 (m, 20H)

### Example 10:

### Compound 10

In a flask connected to an oil bubbler, compound **9** (109 mg, 0.2 mmol) was dissolved in 5 mL of anhydrous DCM/MeOH (7:3). The solution was then cooled to -78°C and ozone was bobbled through it. The gas evolution was stopped during 5 min every 10 min. The temperature was maintained between -78°C and -85°C. Despite the persistence of starting material traces, the reaction was stopped after 7h by purging with nitrogen. The solution was stirred for 1 h at room temperature and concentrated under reduced pressure. The residue obtained was directly purified by flash chromatography (hexane/EtOAc 98:2 to 90:10) to give compound **10** (31 mg, 50%).
¹H NMR (CDCl₃) δ : 0.75 (d, 3H, J=6.0), 0.85 (d, 3H, J=6.0), 1.35 (m, 1H), 1.60 (m, 2H), 3.80 (s, 3H), 4.95 (m, 1 H), 5.02 (s, 2H), 5.15 (s, 1 H), 7.23-7.32 (m, 5H).
¹³C NMR (CDCl₃) δ: 21.80, 23.70, 25.50, 40.60, 53.10, 56.40, 68.80, 128.80, 129.20, 136.40, 156.30, 161.10, 168.22, 193.20.

### Example 11:

### Compound 11

Compound **10** (31 mg, 0.1 mmol) was dissolved in MeOH (5 mL) containing 10% Pd on charcoal (100 mg). The mixture was stirred for 24h at room temperature under a hydrogen atmosphere. The catalyst was removed by filtration through Celite and the filtrate concentrated. The crude amine 11 was immediately used in the next step.

### Example 12:

### Compound 12

To a solution of compound **8** (0.1 mmol) and **11** (0.1 mmol) in anhydrous DCE (2 mL) were added DIEA (0.035 mL, 0.2 mmol) and PyBOP (57mg, 0.11 mmol). The mixture was stirred for 24h at room temperature and 5 mL of water were added. The organic phase was washed with 5% aqueous HCl solution followed by saturated NaHCO₃ before to be dried over MgSO₄. Filtration and concentration under reduced pressure gave a crude product which was purified by flash chromatography (hexane/EtOAc from 9:1 to 7:3) to give compound **12** (0.02 mmol, 20%).
¹H NMR (CDCl₃) δ: 0.75-0.90 (m, 6H), 1.12 (s, 9H), 1.38 (s, 9H), 1.40-1.65 (m, 3H), 1.65-2.10 (m, 3H), 2.10-2.45 (m, 4H), 3.62 (s, 3H), 3.68 (m, 2H), 3.72 (s, 3H), 4.24 (m, 2H), 4.42 (m, 2H), 4.57 (m, 1 H), 5.30 (m, 1 H), 6.76 (s, 1 H) SM (GT, FAB⁺): 588 (M+1H)⁺

### Example 13:

### Compound 13

Compound **12** (43 mg, 0.07 mmol) was dissolved in a mixture of MeOH (0.5 mL) and 1 N aqueous NaOH solution (0.15 mL). The mixture was stirred for 1 h at room temperature, extracted with Et₂O and then acidified (pH=2) by the addition of 5% aqueous HCl solution. The acidic aqueous phase was extracted with EtOAc and the combined organic phases washed with brine and dried over MgSO₄. The crude product was purified by precipitation (addition of hexane in a DCM solution) to give compound **13** (22 mg, 56%) as a white powder.
¹H NMR (CDCl₃) δ: 0.60-0.70 (m, 6H), 0.96 (s, 9H), 1.05 (m, 1H), 1.22 (s, 9H), 1.20-1.55 (m, 3H), 1.60-2.04 (m, 4H), 2.13-2.30 (m, 2H), 3.34 (m, 1 H), 3.62 (m, 1 H), 4.00-4.21 (m, 4H), 4.42 (m, 1 H), 5.53 (m, 1 H), 6.96 (m, 1 H) SM (GT, FAB⁺): 559 (M+1H)⁺

### Example 14:

### Compound 14

Compound **9** (483 mg, 0.90 mmol) was dissolved in MeOH (30 mL) and 10% Pd on charcoal (1.4 g) was added carefully at room temperature followed by ammonium formate (2.1g). After 10 min carbon dioxide evolution was observed. When TLC indicated complete conversion (30 min), the catalyst was removed by filtration and washed thoroughly with EtOAc. Combined organic phases were washed with water followed by brine and dried over MgSO₄. Concentration under reduced pressure gave **14** which was used without further purification.

### Example 15:

### Compound 15

To a solution of compound **8** (0.9 mmol) and compound **14** (0.9 mmol) in anhydrous DCE (4 mL) were added DIEA (0.31 mL, 1.80 mmol) and PyBOP (515 mg, 0.99 mmol). The mixture was stirred for 24h at room temperature and 10 mL of water were added. The organic phase was washed with 5% aqueous HCl solution followed by saturated NaHCO₃ before to be dried over MgSO₄. Filtration and concentration under reduced pressure gave a crude product which was purified by flash chromatography (hexane/EtOAc from 9:1 to 1:1) to give compound **15** (0.48 mmol, 54%).
¹H NMR (CDCl₃) δ: 0.88 (d, 3H, J=5.9), 0.92 (d, 3H, J=5.9), 1.12 (s, 9H), 1.30 (m, 1 H), 1.33 (s, 9H), 1.50-2.00 (m, 7H), 2.30 (m,2H), 3.35 (m, 1 H), 3.60 (s, 3H), 3.75 (m, 1 H), 4.40 (m, 2H), 5.00 (m, 1 H), 5.25 (m, 1 H).

### Example 16:

### Compound 16

In a flask connected to an oil bubbler, compound **15** (185 mg, 0.22 mmol) was dissolved in 6 mL of anhydrous DCM/MeOH (7:3). The solution was then cooled to -78°C and ozone was bubbled through it. The gas evolution was stopped during 5 min every 10 min. The temperature was maintained between -78°C and -85°C. Despite the persistence of starting material traces, the reaction was stopped after 7h by purging with nitrogen. The solution was stirred for 1 h at room temperature and concentrated under reduced pressure. The residue obtained was directly purified by flash chromatography (hexane/EtOAc 98:2 to 90:10) to give compound **16** (82 mg, 64%).
¹H NMR (CDCl₃) δ: 0.86 (d, 6H, J=5.9), 1.11 (s, 9H), 1.20 (t, 1H, J=5.9), 1.36 (s, 9H), 1.50-2.10 (m, 6H), 2.45 (m, 3H), 3.40 (m, 1 H), 3.61 (s, 3H), 3.70 (m, 1 H), 3.80 (s, 3H), 4.35-4.45 (m, 2H), 4.60 (dd, 1 H, J=3.0, 8.5), 5.00 (m, 1H), 5.25 (d, 1 H).
¹³C NMR (CDCl₃) δ: 192.07 (ketone).
SM (GT, FAB⁺): 586 (M+1H)⁺

### Example 17:

### Compound 17

Compound **16** (50 mg, 0.08 mmol) was dissolved in a mixture of MeOH (1 mL) and 1 N aqueous NaOH solution (0.33 mL). The mixture was stirred for 1 h at room temperature, extracted with Et₂O and then acidified (pH=2) by the addition of 5% aqueous HCl solution. The acidic aqueous phase was extracted with EtOAc and the combined organic phases washed with brine and dried over MgSO₄. The crude product was purified by precipitation (addition of hexane in a DCM solution) to give compound **17** (25 mg 52%) as a white powder.
¹H NMR (CDCl₃) δ 1.10 (s, 9H), tBu) and 1.35 (s, 9H, tBu); disappearance of OMe peaks.
SM (GT, FAB⁺): 558 (M+1H)⁺

### Example 18 : primary cultures of human hepatocytes represent an in vitro model suitable for studying HCV infection

### 1-1- Description of the model of study:

Interestingly, HCV was shown to be able to replicate in primary cultures of human hepatocytes infected *in vitro*, thanks to the activity of the viral RdRp (12).

The basic protocol was as follows: 1) human hepatocytes extracted from liver lobectomies of HCV-negative patients were cultured in the long-term culture system mentioned above; 2) cells were then infected *in vitro* with an aliquot of serum from HCV-positive patients; 3) after a few days of culture (1 to 14 days), cellular RNA was extracted and analyzed for HCV RNA (+) and (-) strands using a highly specific rtTh. The implemented RT-PCR method is as described in Sambrook and Russel, 2001, Molecular cloning: a laboratory manual, 3^{rd} ed., Cold Spring Harbor Laboratory Press, Cold Spring Harbor, N.Y..

A more detailed description of the experimental procedure used in the context of the present invention is given hereunder for one chemical compound (example 5). Of course, the procedure can differently be used for each compound described according to the present invention.

Three cultures of HCV-negative hepatic cells were used, named FT200, FT201, and FT203. These cells were extracted from metastasis of colic tumors. (for the same tissue in three different patients).

Once in culture, hepatocytes were infected using a serum (S183) of genotype 3 from HCV-positive patients. Infection was performed in the presence or the absence of a compound to be tested (here, compound of example 5), at concentrations of 0.1, 1, and 10 µM.

Cultures were maintained during 5 days with or without M107 treatment.

Cells were then washed and harvested.

RNA was extracted and analyzed using: first, rtTh RT-PCR and agarose gel electrophoresis (control of the presence of the replicative strand); and second, quantitative RT-PCR using (a) the genomic strand of HCV, and (b) GAPDH RNA /GAPDH RNA as an internal control.

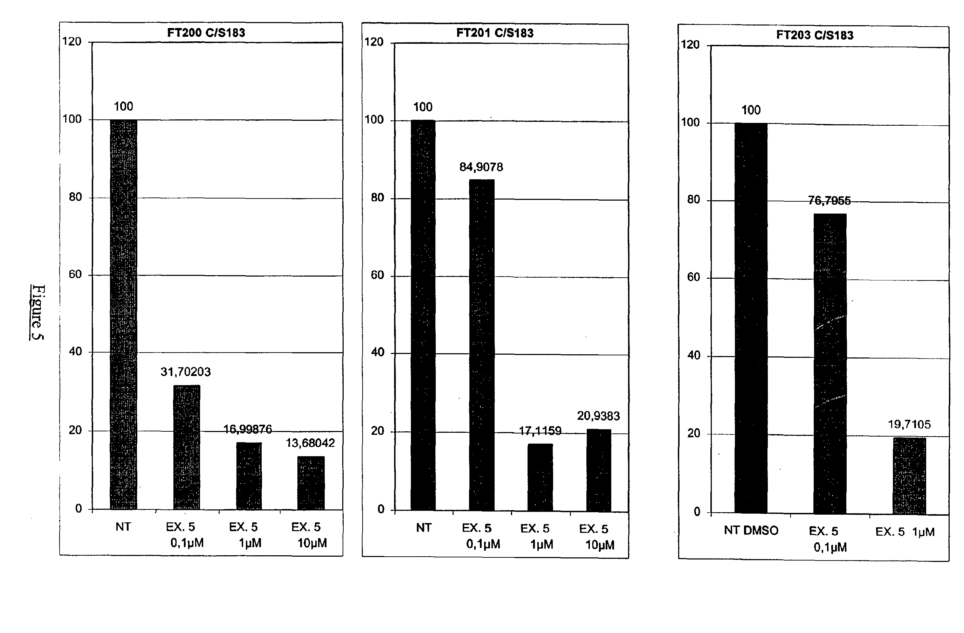

The results, illustrated in Figure 5, were expressed as the ratio HCV RNA/GAPDH RNA, said ratio being normalized to 100 for cells infected but untreated with compound 5.

### 1-2- Results:

As an example, a significant inhibitory effect of the compound of example 5 was obtained using 1 µM of said compound (see figure 5).

Thus, the compounds of the invention were found to have a strong inhibitory effect on the HCV in human hepatocytes.

### References:

1. Alter, H.J. & Seeff, L.B. Recovery, persistence, and sequelae in hepatitis C virus infection: a perspective on long-term outcome. *Semin Liver Dis* **20**, 17-35. (2000).
2. Major, M.E. & Feinstone, S.M. The molecular virology of hepatitis C. *Hepatology* **25**, 1527-38. (1997).
3. Tu, H. et al. Hepatitis C virus RNA polymerase and NS5A complex with a SNARE-like protein. *Virology* **263**, 30-41. (1999).
4. Ishido, S., Fujita, T. & Hotta, H. Complex formation of NS5B with NS3 and NS4A proteins of hepatitis C virus. *Biochem Biophys Res Commun* **244**, 35-40. (1998).
5. Shimizu, Y.K. & Yoshikura, H. Multicycle infection of hepatitis C virus in cell culture and inhibition by alpha and beta interferons. *J Virol* **68**, 8406-8. (1994).
6. Lanford, R.E., Sureau, C., Jacob, J.R., White, R. & Fuerst, T.R. Demonstration of in vitro infection of chimpanzee hepatocytes with hepatitis C virus using strand-specific RT/PCR. *Virology* **202**, 606-14. (1994).
7. Negro, F. et al. Detection of intrahepatic hepatitis C virus replication by strand- specific semi-quantitative RT-PCR: preliminary application to the liver transplantation model. *J Hepatol* **29**, 1-11. (1998).
8. Weiner, A.J. et al. Variable and hypervariable domains are found in the regions of HCV corresponding to the flavivirus envelope and NS1 proteins and the pestivirus envelope glycoproteins. *Virology* **180**, 842-8. (1991).
9. Martell, M. et al. Hepatitis C virus (HCV) circulates as a population of different but closely related genomes: quasispecies nature of HCV genome distribution. *J Virol* **66**, 3225-9. (1992).
10. McHutchison, J.G. et al. Interferon alfa-2b alone or in combination with ribavirin as initial treatment for chronic hepatitis C. *N Engl J Med* **339**, 1485-92. (1998).
11. Poynard, T. et al. Randomised trial of interferon alpha2b plus ribavirin for 48 weeks or for 24 weeks versus interferon alpha2b plus placebo for 48 weeks for treatment of chronic infection with hepatitis C virus. *Lancet* **352,** 1426-32. (1998).
12. Fournier, C. et al. In vitro infection of adult normal human hepatocytes in primary culture by hepatitis C virus. *J Gen Virol* **79**, 2367-74. (1998).
13. Piccininni, S. et al. Modulation of the hepatitis C virus RNA-dependent RNA polymerase activity by the NS3 helicase and the NS4B membrane protein. J *Biol Chem* 2002 Sep 15, (epub ahead of print)

## Claims

1. A compound having the general structure (I) or (II) as follows : 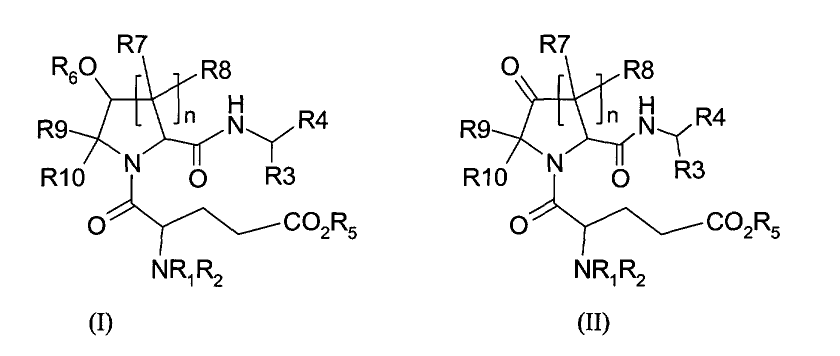 wherein R1 represents a hydrogen atom; R2 represents an amine protecting group; R3 represents an alkyl group having from 1 to 10 carbon atoms inclusive or an amino acid side chain; R4 represents an acid group, an ester group, an alkanoyl group, a keto-acid, a keto-ester, a keto-amide or a α-hydroxy-keto derivative; R5 represents a hydrogen atom, an alkyl group having from 1 to 10 carbon atoms inclusive or a carboxy protecting group; R6 represents a hydrogen atom or an alkyl group having from 1 to 10 carbon atoms inclusive; R7 and R8 are the same or different and each represents a hydrogen atom, an alkyl group having from 1 to 10 carbon atoms inclusive or a cycloalkyl group having from 1 to 10 carbon atoms inclusive, including a cyclohexyl derivative; R9 and R10 are the same or different and each represents a hydrogen atom or an alkyl group having from 1 to 10 carbon atoms inclusive; n is 1 or 2,
their tautomers, optical and geometrical isomers, racemates, salts, hydrates and mixtures thereof.

2. A compound according to claim 1, wherein the compound corresponds to the following general formula (la) or (Ila) :

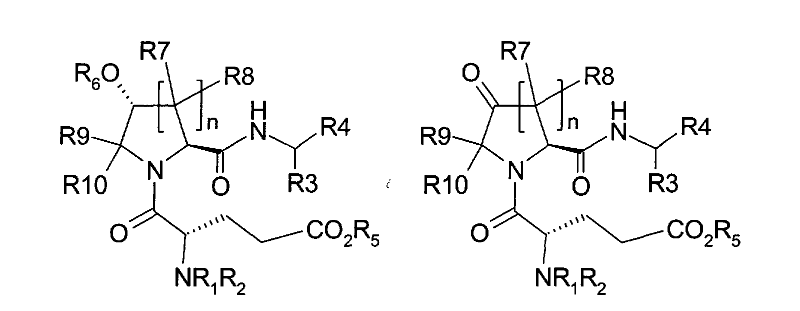

3. Compounds according to claim 1 or 2, wherein the compound corresponds to general formula (I) or (II) wherein :
R2 stands for benzyloxycarbonyl (Cbz) or t-butyloxycarbonyl (Boc) groups and/or R3 stands for -CH₂-CF₃ or -CH₂-CHMe₂; and/or R4 is acid, -CHOHCOR, with R is OH or an alkoxy group (preferably methoxy or ethoxy), keto-acid, keto-ester (preferably -COCOOMe or-COCOOEt) ; and/or R5 is H or benzyl ; and/or R6 stands for t-butyl ; and/or R7 is H ; and/or R8 is H ; and/or R9 is H; and/or R10 is H and/or n = 1.

4. A compound of formula (I) or (II) as defined in claim 1, which is selected in the group consisting of compounds of formula (I).

5. A compound of formula (I) as defined in claim 1, which is selected in the group consisting of :

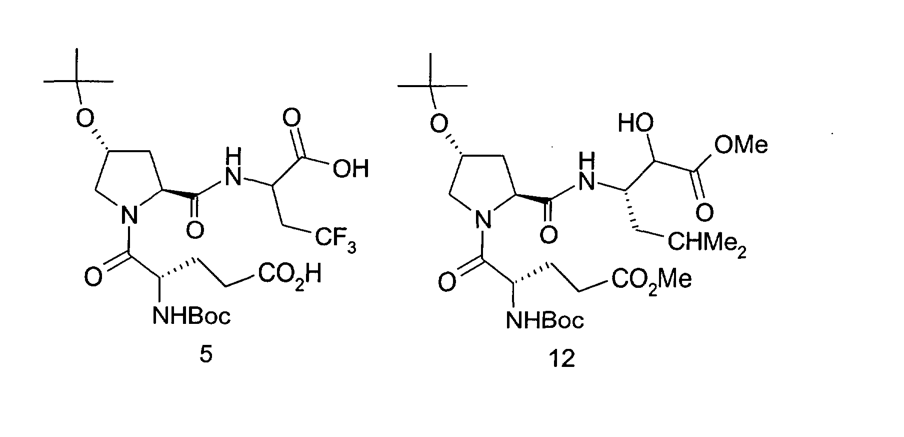

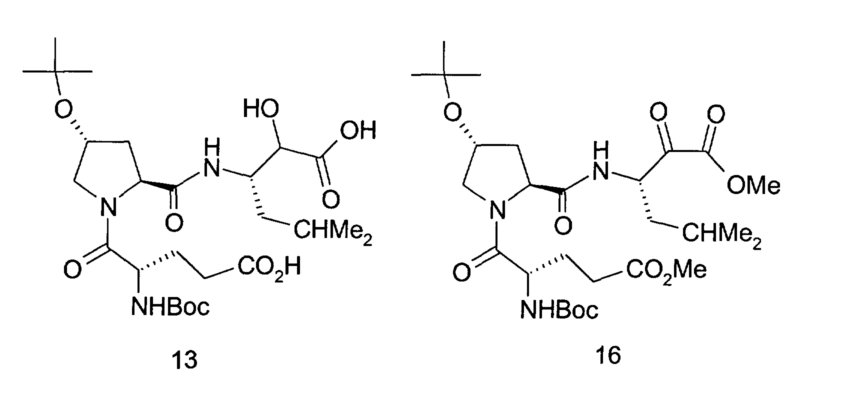

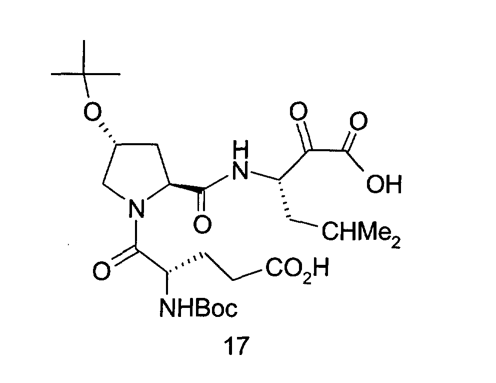

6. A compound corresponding to the following formula (III) : 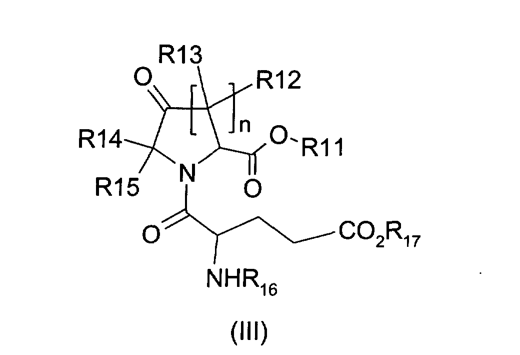 wherein R12, R13, R14 and R15, independently from each other, represents a hydrogen atom, an alkyl, an alkoxy group, a cycloalkyl, an acid group, an ester group, an alkanoyl group, a keto-acid, a keto-ester, a keto-amide or a α-hydroxy-keto group; R16 represents an amine protecting group; R11 and R17, independently from each other, represents a hydrogen atom, an alkyl group or a cycloalkyl group, including cyclohexyl group; and n is 1 or 2, its tautomers, optical and geometrical isomers, racemates, salts, hydrates and mixtures thereof.

7. A compound according to the preceding claim, wherein it presents the following formula (IIIa) :

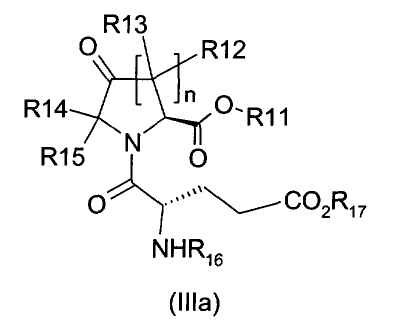

8. A compound according to claim 6 or 7, wherein it corresponds to compounds of formula (III) wherein R12, R13, R14 and R15, independently from each other, represents a hydrogen atom, an alkyl, an alkoxy group, or a cycloalkyl group, and preferably a hydrogen atom.

9. A compound according to the preceding claim which has the following formula:

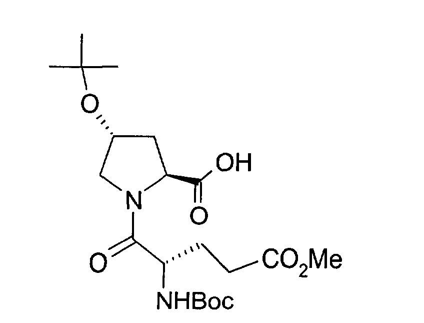

10. A compound according to claim 6 or 7, wherein it corresponds to compounds of formula (III) wherein at least one of R12, R13, R14 and R15 represents an acid group, an ester group, an alkanoyl group, a keto-acid, a keto-ester, a keto-amide or a α-hydroxy-keto group.

11. A compound according to one of claims 6-10, useful as an intermediate compound to prepare a compound of formula (I) or (II) as defined in claims 1-4 or as an active pharmaceutical ingredient, such as an antiviral agent (antiviral HCV agent).

12. A pharmaceutical composition comprising at least one compound as defined in any of the preceding claims and a pharmaceutically acceptable vehicle or support.

13. A pharmaceutical composition according to the preceding claim, said composition further comprising at least one immunomodulatory agent, other antiviral agent, other inhibitor of hepatitic C protease; inhibitor of other targets in the HCV life cycle, or combinations thereof.

14. A pharmaceutical composition according to claim 12 or 13, useful for treating a disease related to an infection by a virus (preferably flavivirus, such as dengue virus, yellow fever virus or HCV), bacteria or pathogen dependent upon a serine protease for proliferation

15. A pharmaceutical composition according to claim 12 or 13, useful for treating HCV infection and the like.

16. A pharmaceutical composition according to claim 12 or 13, useful for treating hepatitis C virus infection and complications thereof, in particular chronic hepatitis, cirrhosis or hepatocellular carcinoma and extrahepatic manifestation.

17. Use of an effective amount of at least one compound of formula (I), (II) or (III) as defined in any of the claims 1-10 for the preparation of pharmaceutical composition intended for the treatment of a disease associated with an infection by a virus (preferably flavivirus, such as dengue virus, yellow fever virus or HCV), bacteria or pathogen dependent upon a serine protease for proliferation.

18. Use of an effective amount of at least one compound of formula (I), (II) or (III) as defined in any of the claims 1-10 for the preparation of pharmaceutical composition intended for the treatment of a disease associated with HCV infection.

19. A method of evaluating the modulation properties of test compounds towards NS3 serine protease, particularly HCV NS3 serine protease, said method implementing *in vitro* primary cultures of human hepatocytes and compounds as defined in any of the claims 1-10.

20. A method for screening and/or characterizing compounds that present antiviral activity, in particular antiviral HCV activity, by implementing *in vitro* primary cultures of human hepatocytes and compounds as defined in any of the claims 1-10.

21. A method according to the preceding claim, said method comprising the following steps :
c) contacting a test compound with the *in vitro* primary cultures of human hepatocytes described herein in presence of HCV or active part thereof, and
d) determining the antiviral activity of the test compound in comparison with the antiviral activity of one of the compounds as defined in any one of claims 1-10.

22. Use of at least one compound as defined in any of the preceding claims 1-10 as an agent to treat or prevent viral contamination of materials.
